Europäisches Patentamt

European Patent Office

Office européen des brevets

(19)

(11) Numéro de publication: **0 331 584**
**A1**

# (12) DEMANDE DE BREVET EUROPEEN

(21) Numéro de dépôt: **89400596.6**

(51) Int. Cl.⁴: **A 61 K 6/10**

(22) Date de dépôt: **03.03.89**

(30) Priorité: **03.03.88 FR 8802723**

(43) Date de publication de la demande:
**06.09.89 Bulletin 89/36**

(84) Etats contractants désignés:
**AT BE CH DE ES FR GB GR IT LI LU NL SE**

(71) Demandeur: **SANOFI**
**40, Avenue George V**
**F-75008 Paris (FR)**

(72) Inventeur: **Ridoux, Claude**
**Chemin des Busclats**
**F-84800 Isle sur la Sorgue (FR)**

(74) Mandataire: **Gillard, Marie-Louise et al**
**Cabinet Beau de Loménie 55, Rue d'Amsterdam**
**F-75008 Paris (FR)**

(54) Composition pulvérulente à base d'alginate pour empreintes dentaires.

(57) Composition pulvérulente pour empreintes dentaires contenant au moins un alginate hydrosoluble, un agent gélifiant, un régulateur de gélification et des charges revêtues d'une gélatine hydrolysée.

EP 0 331 584 A1

## Description

### COMPOSITION PULVERULENTE A BASE D'ALGINATE POUR EMPREINTES DENTAIRES.

La présente invention concerne une composition pulvérulente pour la réalisation d'empreintes dentaires, à base d'alginate, dont les caractéristiques de mouillabilité, de fluidité et pulvérulence sont améliorées par rapport aux produits classiques.

On utilise couramment pour faire des empreintes dentaires, des produits composés essentiellement d'au moins un alginate hydrosoluble, d'un agent gélifiant des solutions d'alginates, d'un agent régulateur de la prise en gel, et de charges minérales en poudre.

L'alginate hydrosoluble peut être un alginate alcalin, un alginate d'ammonium, d'alcanolamine ou un mélange de ceux-ci ; l'agent gélifiant peut être un sel métallique: un sel métallique d'acide gras comme un oléate de calcium, un laurate de zinc, un stéarate de fer ou un sel métallique d'acide minéral, tel qu'un sulfate de fer ou zinc ou, et de préférence, un sulfate de calcium hydraté ; l'agent régulateur de la prise en gel peut être choisi parmi les phosphates, pyrophosphates, silicates, carbonates ou citrates alcalins et leurs mélanges. Les charges qui sont introduites dans le mélange pour améliorer sa consistance et sa résistance, sont en général des terres de diatomées, de la silice, du talc, de la perlite ou leurs mélanges.

Le mélange poudreux des composants est malaxé avec de l'eau par le praticien juste avant la prise de l'empreinte, qui est effectuée en appliquant la pâte obtenue, à l'aide d'un porte-empreinte sur la zone de la mâchoire du patient dont on veut enregistrer la morphologie, pour préparer une prothèse ou réaliser un modèle ; lorsque la pâte s'est gélifiée, l'empreinte du fait de son élasticité peut être retirée de la bouche pour être coulée en plâtre. Ce modèle de plâtre est utilisé ultérieurement pour préparer une prothèse ou pour faire une étude en orthopédie dentofaciale.

On pourra se référer à l'ouvrage de E.W. SKINNER et RW. PHILIPS - Science des matériaux dentaires - publié chez Julien PRELAT, Paris qui donne la composition type du produit pour empreintes à base d'alginate.

On a aussi proposé l'addition de différents composés à ces compositions classiques pour améliorer les propriétés du produit, et notamment la malléabilité de la pâte par addition de plastifiants comme des alcools, des huiles minérales ou végétales ; ou la précision de l'empreinte par exemple par addition de fluorures, de préférence avec des oxydes ou des hydroxydes alcalins pour améliorer aussi la stabilité de la poudre et la force du gel, comme décrit dans US 3,291,668 ou par addition de polyacrylamide dans le mélange comme décrit dans EP 198131 ou encore par addition de polymères à la pâte avant son utilisation comme décrit dans US 3,620,778.

Des colorants, des aromatisants et des conservateurs peuvent aussi être introduits dans le produit.

Plus récemment, on a proposé des compositions pour empreintes dentaires dont la mise en oeuvre s'accompagne d'un faible dégagement de poussières. En effet, il est fréquent qu'au cours du stockage le mélange devienne hétérogène, les constituants les plus denses ayant tendance à sédimenter dans le fond de la boîte, et le praticien a l'habitude de secouer le récipient avant d'y prélever la quantité de poudre nécessaire à la préparation d'une pâte pour empreinte, ce qui entraîne, à l'ouverture de la boîte, la dispersion dans l'air ambiant de fines particules, ce qui est, sans aucun doute, désagréable pour les personnes présentes dans la pièce, mais peut être aussi toxique.

Ainsi, la demande de brevet EP-A-0058203, décrit une composition pulvérulente pour empreintes dentaires dont une partie au moins des constituants a été revêtue avec un produit facile à mouiller, à disperser ou à dissoudre dans l'eau ; parmi ces produits sont cités des polymères dispersants, tels que la gomme xanthane, les polyalginates alcalins, des ethers et esters de cellulose, et des polymères surfactifs, tels que les polyalkylèneglycols ou même des molécules de faible massemoléculaire, tels que des polyols, des esters de glycérol, des alkanolamines, et des laurylsulfates.

Dans la demande de brevet JP 58-98021, on préconise le revêtement des grains de la poudre avec un surfactif non ionique et un liquide hydrophobe ayant une tension de vapeur inférieure à 3,15 mmHg à 20° C tandis que dans FR-A-2 553 999, on ajoute à la poudre ainsi revêtue de la polyvinylpyrrolidone pour améliorer la stabilité du produit durant le stockage et l'état de surface du moulage en plâtre qui sera préparé à partir de l'empreinte d'alginate.

Dans la demande de brevet EP-A-0217 270, on préconise l'introduction d'une isoparaffine, telle que le 2,2,4,4,6,6,8-heptaméthylnonane ou le 2,2,4,4,6,6,8,8,10-nonaméthylundecane dans un mélange classique pour diminuer la libération de poussières volatiles.

Enfin dans la demande de brevet DE-A-35 11 721, on préconise la granulation de la totalité du mélange avec un polymère tel que l'hydroxypropylcellulose ou la polyvinylpyrrolidone.

Les produits de la présente invention ne libèrent pas de poussière lorsqu'ils sont mis en oeuvre ; par ailleurs, ils s'écoulent bien et ne sont pas collants comme peuvent l'être les produits traités par des liquides et ils sont mouillés facilement par l'eau, contrairement aux produits traités par des composés hydrophobes ou granulés, ce qui rend la préparation et l'homogènéisation de la pâte plus facile.

Selon la présente invention, les charges introduites dans un produit pour empreintes dentaires à base d'alginate de composition classique, sont préalablement revêtues d'une gélatine hydrolysée, hydrosoluble.

Les charges sont revêtues d'une pellicule de gélatine hydrolysée, par pulvérisation, par immersion ou par aspersion de la poudre de charges avec une solution aqueuse de gélatine hydrolysée dans une turbine à dragéifier, un mélangeur planétaire, un mélangeur granulateur, un granulateur à tamis ou en lit d'air fluidisé par exemple dans un granulateur pulvérisateur, puis les grains ainsi pelliculés sont séchés par action d'un courant d'air chaud, à une température comprise entre 20° C et 80° C, dans un appareil convenable, tel qu'un tunnel de séchage ou dans l'appareil où a eu lieu le pelliculage s'il s'agissait d'une turbine à dragéifier ou d'un appareil à lit d'air fluidisé.

La pellicule de gélatine hydrolysée représente de 1 à 10 % en poids du poids des charges et plus particulièrement de 3 à 6 %.

La gélatine hydrolysée est préparée par action d'un acide, d'une base, de la chaleur ou d'une enzyme sur une gélatine classique résultant de l'hydrolyse acide ou alcaline du collagène présent dans les os, les peaux ou les tendons des bovins, porcins ou ovins. La gélatine de départ a une masse moléculaire généralement comprise entre 60 000 et 300 000, tandis que la gélatine hydrolysée a une masse moléculaire moyenne en poids comprise entre 500 et 30 000 et de préférence entre 5 000 et 10 000. Cette gélatine hydrolysée est soluble dans l'eau, à la température ambiante, c'est-à-dire vers 20° C et ne présente pas de force en gel mesurable dans les conditions définies par les "British Standards", à la concentration de 6,67 % (p/v) dans l'eau après 17 heures à 10° C.

Les procédés d'hydrolyse de la gélatine sont bien connus de l'homme de l'art ; on pourra se référer notamment aux brevets FR-A-2 099 777 et US 4,130,555 ou aux documents publiés par NOVO (Danemark) et notamment à NOVO Enzyme Information réf : 163b.

Des gélatines hydrolysées sont disponibles sur le marché ; on peut notamment citer la gélatine ayant la référence SPG ou la référence DSF, commercialisée par MERO ROUSSELOT SATIA devenu SANOFI BIO INDUSTRIE (France) et la gélatine dénommée Crotein A, commercialisée par CRODA (Grande-Bretagne).

Dans ce qui suit, on décrit des exemples de mise en oeuvre de l'invention, et on compare les résultats obtenus avec ceux des compositions n'ayant pas les caractéristiques de l'invention.

## EXEMPLE 1

La charge utilisée est une terre de diatomée, dénommée Clarcel, elle est revêtue de la gélatine hydrolysée, commercialisée par la Société MERO ROUSSELOT SATIA sous la référence SPG.

Dans un granulateur à lit d'air fluidisé de type Uniglatt commercialisé par Glatt (RFA), on introduit 400 g de Clarcel et 320 ml d'une solution aqueuse à 5 % (p/v) de gélatine SPG (MERO ROUSSELOT SATIA). La masse de poudre étant mise en suspension dans l'air, on pulvérise sous pression, la solution de liant sur le lit de poudre fluidisé.

On obtient ainsi une poudre granuleuse de Clarcel pelliculé, contenant 4 % de gélatine, qui s'écoule librement et dont les caractéristiques, comparées à celles du produit de départ, figurent dans le tableau I.

### TABLEAU I

|  | Clarcel | Clarcel pelliculé |
|---|---|---|
| Volume apparent : | 4 m³/kg | 5,36 m³/kg |
| Densité apparente : | 0,25 kg/m³ | 0,186 kg/m³ |
| Granulométrie : |  |  |
| < 500 μm | 0,16 % | 1,4 % |
| < 250 μm | 1,1 % | 10,64 % |
| < 100 μm | 24,2 % | 61,54 % |
| < 40 μm | 46,62 % | 23,04 % |
| > 40 μm | 27,92 % | 3,38 % |

La composition pour empreinte est préparée en mélangeant à sec dans un mélangeur rapide à socs, commercialisé par Lödige, les produits suivants :

| Clarcel pelliculé ... | 64,2 g |
|---|---|
| Alginate d'algue laminaire sous forme potassique ... | 15,1 g |
| Sulfate de calcium, dihydrate ... | 1 g |
| Fluorotitanate de potassium ... | 4,6 g |

La composition est ensuite mise en boîte. A l'ouverture de celle-ci après secouage, il n'y pas de libération de poudres dans l'air.

On mélange alors 10 g de ce produit avec 21 ml d'eau, à la spatule, dans un petit bol en plastique semi-rigide ; la poudre est mouillée très rapidement et l'eau s'incorpore facilement, la pâte est prête en 30 secondes et elle prend après 3 minutes, à 20° C.

Le mélange poudreux, préparé dans les mêmes conditions mais avec du Clarcel non traité, libère des particules dans l'air après secouage de la boîte et la préparation de la pâte est plus difficile : la poudre est moins bien mouillée, et l'incorporation de la totalité de l'eau dans la pâte plus longue.

Les deux produits donnent le même temps de prise en gel et le gel obtenu permet, dans les deux cas, de reproduire des détails très fins de l'ordre de 35 m.

Un autre mélange poudreux a été préparé dans les mêmes conditions, mais avec du Clarcel pelliculé avec une gélatine ordinaire, ayant une force en gelée de 80 blooms mesurée selon les British Standards. Le mélange secoué ne libère pas de poussières, mais on ne peut préparer avec cette composition une pâte convenant à la prise d'empreintes : les grains sont difficiles à mouiller et ne s'écrasent pas à la spatule et l'on obtient finalement un gel granuleux.

## EXEMPLE 2

On utilise la même charge pelliculée qu'à l'exemple 1, mais pour préparer une poudre pour empreintes donnant une pâte fluide, applicable à la seringue de répartition :

Le mélange est constitué de :

| | |
|---|---|
| Clarcel granulé ...... | 63 g |
| Alginate d'algue laminaire sous forme potassique ...... | 6,5 g |
| Alginate de cloustonii sous forme sodique ...... | 6,5 g |
| Sulfate de calcium ...... | 3,7 g |
| Fluorotitanate de potassium ...... | 2 g |
| Oxyde de magnésium ...... | 1 g |

Ce mélange ne libère pas de poussières au secouage.

On prépare une pâte fluide avec 7 g de mélange poudreux et 21 ml d'eau.

Cette pâte prend en gel en 4 minutes à 20° C. Le mélange préparé avec du Clarcel ordinaire libère de la poussière et il est plus difficile de préparer une pâte fluide, homogène qu'avec le mélange selon l'invention. Le temps de prise, la finesse du gel obtenu et la possibilité de reproduire de fins détails ne sont pas altérés avec le produit selon l'invention.

## Revendications

1. Composition pour empreintes dentaires contenant au moins un alginate hydrosoluble, un agent gélifiant, un régulateur de gélification et des charges minérales, caractérisée en ce que les charges minérales sont revêtues avec une gélatine hydrolysée.

2. Composition selon la revendication 1, caractérisée en ce que les charges sont revêtues de 1 à 10 % en poids de gélatine hydrolysée.

3. Composition selon l'une des revendications 1 ou 2, caractérisée en ce que le pelliculage est effectué dans un granulateur à lit d'air fluidisé.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 572 931 (SANKIN)<br>* Revendications; page 3, lignes 10-20 *<br>--- | 1-3 | A 61 K    6/10 |
| A,D | WO-A-8 200 650 (DENTSPLY)<br>* Revendications * & EP-A-58 203<br>----- | 1-3 | |

DOMAINES TECHNIQUES RECHERCHES (Int. Cl.4)

A 61 K
C 08 L

Le présent rapport a été établi pour toutes les revendications

| Lieu de la recherche | Date d'achèvement de la recherche | Examinateur |
|---|---|---|
| LA HAYE | 18-05-1989 | COUSINS-VAN STEEN G.I.L. |